Europäisches Patentamt

European Patent Office

Office européen des brevets

(11) Veröffentlichungsnummer: **0 314 021**
**A2**

## (12) EUROPÄISCHE PATENTANMELDUNG

(21) Anmeldenummer: 88117621.8

(22) Anmeldetag: 22.10.88

(51) Int. Cl.⁴: **A61B 17/60**

(30) Priorität: 26.10.87 DE 3736136
18.06.88 DE 3820646

(43) Veröffentlichungstag der Anmeldung:
03.05.89 Patentblatt 89/18

(84) Benannte Vertragsstaaten:
**AT CH DE ES FR IT LI**

(71) Anmelder: **Howmedica GmbH**
**Professor-Küntscher-Str. 1-5**
**D-2314 Schönkirchen ü. Kiel(DE)**

(72) Erfinder: **Rogge Dirk H. Dr.**
**Hohe-Lieth-Weg 6**
**D-2854 Loxstedt-Duering(DE)**

(74) Vertreter: **Dipl.-Ing. H. Hauck Dipl.-Ing. E.**
**Graalfs Dipl.-Ing. W. Wehnert Dr.-Ing. W.**
**Döring**
**Neuer Wall 41**
**D-2000 Hamburg 36(DE)**

(54) **Vorrichtung zum Fixieren von Knochenteilen.**

(57) Vorrichtung zum Fixieren von Knochenteilen, mit mindestens einer rohrförmigen Trägerstange, bei der mindestens die Innenkontur im Querschnitt unrund ist, mit mindestens einer an das Querschnittsprofil der Trägerstange angepaßten Haltevorrichtung, die längsverschieblich, jedoch drehfest an der Trägerstange geführt und mit mindestens einer Feststellschraube in einer beliebigen Position einer Trägerstange festlegbar ist, und mit mindestens einer an der Haltevorrichtung angebrachten Klemmvorrichtung, in der eine längliche Knochenschraube oder ein -stift axial verschiebbar geführt und in einer gewünschten axialen Position klemmend festlegbar ist, wobei die Haltevorrichtung als an das Innenprofil der Trägerstange angepaßtes Gleitstück ausgebildet ist, das in der Trägerstange frei längsverschieblich geführt ist, die Feststellschraube in einer Gewinde-bohrung der Trägerstange sitzt und die Trägerstan-ge mit Längsschlitzen versehen ist zum Herausfüh-ren einer im Gleitstück festklemmbaren Knochenschraube oder eines Schaftes, der seinerseits die Klemmvorrichtung hält.

Fig. 1

## Vorrichtung zum Fixieren von Knochenteilen

Die Erfindung bezieht sich auf eine Vorrichtung zum Fixieren von Knochenteilen nach dem Oberbegriff des Patentanspruchs 1.

Die Erfindung bezieht sich insbesondere auf einen sogenannten Fixateur externe, bei dem die Halterung für die in die Knochenfragmente eingeschraubten Knochenschrauben, Knochenstiften oder dergleichen, zum Beispiel sogenannte Schanz'sche Schrauben, außerhalb des betreffenden Körperteils angeordnet ist.

Unter der Bezeichnung "Monofixateur" der Firma Orthopaedia GmbH, Kiel ist eine derartige Vorrichtung bekanntgeworden, bei der auf einer hohlen Vierkantstange Klemmschellen aufschiebbar sind, die mit Hilfe von Feststellschrauben in einer beliebigen Position auf einer derartigen Trägerstange feststellbar sind. Von der Klemmschelle begrenzt wird eine Haltebacke, die ebenfalls zusammen mit der Klemmschelle auf die Trägerstange aufgeschoben wird. Sie weist eine Bohrung auf zur Aufnahme einer Knochenschraube, die mit Hilfe einer weiteren Feststellschraube in der Bohrung fixiert werden kann. Es ist auch bekannt, mehrere derartige Trägerstangen durch ein Gelenk miteinander zu verbinden. Der bekannte Fixateur hat den Vorteil, daß er auch zur dynamischen Versorgung verwendet werden kann. Durch Lösen der Feststellschrauben für die Klemmschelle kann diese sich entlang der Trägerstange bewegen, ist jedoch gegen Rotation und Kippbewegungen gesichert. Nachteilig ist, daß im Hinblick auf die Schanz'schen Schrauben relativ geringe Bewegungsmöglichkeit besteht. Außerdem beanspruchen die Klemmschellen und Haltebacken verhältnismäßig viel Platz. Aus der EP-0 153 546 ist ein Fixateur bekanntgeworden, bei dem Trägerteile über eine Doppelgelenkverbindung miteinander verbindbar sind. Die Schanz'schen Schrauben sind mit Hilfe von festklemmbaren Kugeln begrenzt verschwenkbar, jedoch in ihrem Abstand auf dem Trägerteil nicht veränderbar. Eine Längsverschiebung der Knochenteile bzw. der Träger stangen zueinander erfolgt innerhalb des Verbindungsgliedes mittels einer Gewindespindel. Stimmt die Längsachse des Verbindungsgliedes aufgrund der notwendigen Ausrichtung nicht mit der Längsachse des Knochens überein, so findet eine Längsverschiebung nicht in der Achse des Knochens statt, sondern nur in der des Verbindungsglieds. Dadurch kann es zu einer Fehlausrichtung der Knochenfragmente kommen. Außerdem ist keine passive Dynamisierung des Fixateurs möglich.

Bei einem Fixateur nach der US-PS 4 312 336 läßt ein Verbindungsglied zwischen den Tragerteilen zwar eine Dynamisierung zu, die Längsverschiebung ist jedoch ebenfalls nicht notwendigerweise entlang einer Achse parallel zur Knochenachse, so daß eine Fehlausrichtung bzw. -belastung zu befürchten ist.

Aus der DE-A-3 701 533 ist ein Fixateur bekanntgeworden, bei dem Trägerstangenteile über ein Doppel-Kugelgelenk miteinander verbindbar sind. Die Kugelgelenke können mit Hilfe von Feststellschrauben in einer gewünschten Position festgestellt werden. Die Trägerstangen weisen längliche durchgehende Schlitze auf, in denen sogenannte Kulissensteine gleiten, die als Spindelmuttern ausgeführt sind und die auf Gewindespindeln sitzen. Der nach außen stehende Abschnitt der Kulissensteine nimmt einen oder zwei Schanz'sche Schrauben auf. Dieser bekannte Fixateur ermöglicht überhaupt keine Längsverschiebung der Trägerstangen oder der Schanz'schen Schrauben relativ zueinander zum Zwecke der sogenannten Dynamisierung. Eine Verstellung der Kulissensteine und damit der Schanz'schen Schrauben quer zu ihrer Achse kann nur durch eine Verdrehung der Gewindespindeln erreicht werden. Der bekannte Fixateur baut zwar sehr klein, laßt jedoch nur begrenzte Freiheitsgrade zur Aufnahme der Schanz'schen Schrauben zu.

Der Erfindung liegt daher die Aufgabe zugrunde, eine Vorrichtung zum Fixieren von Knochenteilen gegeneinander zu schaffen, die klein baut, eine einwandfreie dynamische Versorgung weitgehend parallel zur Knochenlängsachse zuläßt und ohne die Notwendigkeit vieler Zusatzteile einen größeren Freiheitsgrad für die Halterung der Knochenschrauben vorsieht.

Diese Aufgabe wird erfindungsgemäß gelöst durch die Merkmale des Kennzeichnungsteils des Patentanspruchs 1.

Bei der erfindungsgemäßen Vorrichtung sind die Trägerteile als Rohrprofile ausgeführt, wie bei einer eingangs genannten Vorrichtung (Monofixateur) an sich bekannt. Erfindungswesentlich ist jedoch, daß die Halterung für die Knochenschrauben nicht außen auf das Trägerrohr aufgeschoben ist, sondern in einem Gleitstück angeordnet ist, das in seinem Profil dem Innenprofil des Trägerrohrs angepaßt ist. Das Gleitstück kann daher frei im Trägerrohr verschoben werden. Es wird jedoch durch von außen in das Trägerrohr einschraubbare Feststellschrauben in einer gewünschten Position festgelegt. Das Trägerrohr weist Längsschlitze auf, über die die Knochenschraube nach außen geführt ist und die eine Verschiebung des Gleitstücks auch bei aufgenommener Knochenschraube ermöglicht. Es versteht sich, daß das Gleitstück eine Durchbohrung aufweist, um die Knochenschraube bzw. einen eine Klemmvorrichtung haltenden Schaft aufzunehmen. Im einfachsten Fall kann das Gleitstück seinerseits eine Feststellschraube aufnehmen, um die Knochenschraube in einer gewünschten axialen Position festzulegen. Die Betätigung der Feststellschraube erfolgt ebenfalls über einen Schlitz im Trägerrohr. Es sind jedoch auch weitere Halterungsmöglichkeiten für die Knochenschraube oder den Schaft möglich, worauf weiter unten eingegangen wird.

Die Trägerstange oder das Trägerrohr ist vorzugsweise als quadratisches Vierkantrohr ausgebildet, wobei jedoch die Innen- und/oder Außenkanten abgeschrägt sind. In die derart angefasten Kanten lassen sich leichter Gewindebohrungen einbringen für die Aufnahme von Stellschrauben. Ein besonderer Vorteil der Erfindung besteht darin, daß der die Klemmvorrichtung haltende Schaft quer zur Längsachse der Trägerstange verschoben und variabel fixiert werden kann. Zusammen mit einer um zwei Achsen verstellbaren Klemmvorrichtung am Schaftende resultiert hierdurch die Möglichkeit, die Knochenschrauben oder -stifte primär in variablen Positionen in den Knochenfragmenten zu fixieren und erst sekundär die gesamte Vorrichtung nach erfolgter Einrichtung der Fragmente oder zum Zwecke der Einrichtung anzubringen. Vorzugsweise weisen alle Seitenflächen des Trägerrohrs Schlitze auf, so daß nicht erforderlich ist, das Trägerrohr in einer bestimmten Orientierung anzusetzen.

Die erfindungsgemäße Ausbildung eines Fixateurs hat den Vorteil, daß sie zu einer sehr geringen Baugröße führt, jedoch eine dynamische Versorgung in der Weise ermöglicht, daß eine Verschiebung der Knochenfragmente ausschließlich in der Achse des Knochens erfolgt, während Rotations- und Kippbewegungen durch die Führung des Gleitstücks im Trägerrohr verhindert werden.

Wird die Knochenschraube lediglich in axialer Richtung beweglich im Gleitstück geführt, reicht es aus, wenn nach einer Ausgestaltung der Erfindung im Gleitstück ein Druckelement angeordnet ist, das mittels einer in einer Gewindebohrung des Gleitstücks sitzenden Feststellschraube gegen eine Knochenschraube anpreßbar ist.

Aus Gewichtsersparnisgründen ist es ohnehin zweckmäßig, das Gleitstück mit einer Längsdurchbohrung zu versehen, die senkrecht auf der Querdurchbohrung steht. Wird eine Nut in die Längsdurchbohrung geformt, kann diese das beschriebene Druckelement führen und drehsicher aufnehmen.

Es ist indessen von Vorteil, wenn die Knochenschraube relativ zum Trägerrohr eine unterschiedliche Winkellage einnehmen kann. Daher sieht eine Ausgestaltung der Erfindung vor, daß im Gleitstück mindestens eine Kugelpfanne geformt ist zur Aufnahme einer Kugel, die in einer Durchbohrung die Knochenschraube aufnimmt. Das Gleitstück ist mit Klemmmitteln versehen zur Festlegung der Kugel in einer eingestellten Lage, wobei der Kugel Feststellmittel zugeordnet sind zur Feststellung der Knochenschraube in einer gewünschten axialen Lage in der Kugel. Das Gleitstück kann nach einer Ausgestaltung der Erfindung zweiteilig ausgeführt sein zur Bildung einer geteilten Kugelpfanne. Die Gleitstückteile können zum Beispiel mit Hilfe mindestens einer Schraube gegeneinander gespannt werden, um eine in der Kugelpfanne aufgenommene Kugel in der Kugelpfanne einzupressen, damit sie ihre Lage beibehält. Alternativ kann das Gleitstück auf einer Seite eine längliche Aussparung aufweisen, in der eine Spannplatte eingelegt ist. Die Spannplatte wird mit mindestens einer Schraube gegen das Gleitstück gespannt, um mindestens eine Kugel in der Kugelpfanne festzulegen.

Wird die Kugel starr ausgeführt, muß die Knochenschraube mit Hilfe einer Feststellschraube in der Durchbohrung der Kugel festgelegt werden. Ist hingegen nach einer Ausgestaltung der Erfindung die Kugel geteilt, kann mit Hilfe eines zweigeteilten Gleitstücks bzw. mit Hilfe der oben angeführten Spannplatte sowohl die Kugel als auch die Knochenschraube in einem festgelegt werden. Die Lagerkugelhälften sind vorzugsweise mit Hilfe einer Ringfeder zusammengehalten, die in eine Nut eingelegt ist, die vorzugsweise in einer Ebene senkrecht zur Durchbohrung verläuft. Die Ringfeder bewirkt ferner, daß die Kugelhälften im entspannten Zustand etwa auf Abstand gehalten sind. Nach einer weiteren Ausgestaltung der Erfindung hat die Ringfeder zwei radial nach außen weisende Vorsprünge, die mit Ausnehmungen im Gleitstück zusammenwirken. Die Ausnehmungen im Gleitstück sind jedoch so auszuführen, daß die Kugel eine begrenzte Verschwenkung vornehmen kann in dem Ausmaß, wie sie erforderlich ist, um den notwendigen Schwenkwinkel für die Knochenschraube zuzulassen.

Es ist nicht notwendig, das Trägerrohr besonders lang auszuführen. Vielmehr können verhältnismäßig kurze Trägerrohre einheitlicher Länge vorgegeben werden, die in Modulbauweise mit Hilfe eines geeigneten Kupplungsstücks miteinander verbunden werden, um die jeweils gewünschte Länge einzustellen. Daher sieht eine Ausgestaltung der Erfindung vor, daß ein an die Innenkontur der Trägerstange angepaßtes Kupplungsstück in das Ende der Trägerstange einsetzbar ist zur starren Verbindung von zwei Trägerstangen miteinander. Das Kupplungsstück kann ebenfalls mit Hilfe der bereits beschriebenen Feststellschrauben für die Gleitstücke an der Trägerstange befestigt werden. Daher ist es besonders vorteilhaft, wenn die Reihe der Gewindebohrungen zur Aufnahme einer Feststellschraube sich bis zu den Enden der Trägerstange erstreckt.

Die Ausbildung der Trägerstange als Trägerrohr ermöglicht nach einer weiteren Ausgestaltung der Erfindung auch das Einsetzen eines Anschlußstücks, das zum Beispiel eine Verbindung mit einem weiteren Trägerrohr ermöglicht. Zu diesem Zweck kann das Anschlußstück am freien Ende eine Gelenkkugel aufweisen, die mit einer klemmend wirkenden Kugelpfanne zusammenwirkt. Die Kugelpfanne kann zum Beispiel in einem Verbindungsteil zwischen den Trägerrohren ausgebildet sein. Das Verbindungsglied oder Verbindungsteil kann nach einer weiteren Ausgestaltung der Erfindung auch zwei hintereinander angeordnete Kugelgelenke aufweisen. Hierdurch ist es möglich, die Trägerrohre gegeneinander parallel zu sich selbst zu versetzen. Die Ausbildung eines derartigen Doppelgelenks ist jedoch aus der bereits erwähnten DE-A-3 701 533 bekanntgeworden. Bei der bekannten Vorrichtung sind indessen die Enden der Trägerstangen als Kugelpfannen ausgeführt, während nach einer Ausgestaltung der Erfindung das Verbindungsglied zwei im Abstand voneinander angeordnete Kugelpfannen aufweist, die mit den Kugeln zusammenwirken, die über Verbindungsstäbe mit den Trägerstangen verbunden sind, beispielsweise über die bereits beschriebenen Anschlußstücke. Das Verbindungsglied kann zum Beispiel aus zwei Abschnitten bestehen, wodurch jeweils zwei Kugelpfannenhälften gebildet sind. Die Abschnitte werden mit Hilfe einer Klemmschraube gegeneinandergepreßt, um die Kugeln in den Pfannen festzulegen.

Nach einer weiteren Ausgestaltung der Erfindung kann eine Kugelpfanne auch so ausgeführt sein, daß an ihrem freien Ende eine Stange angebracht ist. Diese Stange kann ihrerseits als Halterung für Anschlußstücke dienen.

Eine andere Möglichkeit zur Anbringung eines Anschlußstückes besteht erfindungsgemäß darin, daß mit der Kugel eines Kugelgelenks ein Klemmkörper verbunden ist, auf dessen der Kugel abgewandten Seite ein Gewindestift angebracht ist, auf dem Gewindestift ein zweiter ringförmiger Klemmkörper verschiebbar angeordnet ist, beide Klemmkörper mittels einer auf dem Gewindestift geschraubten Mutter gegeneinanderpreßbar sind und die einander zugekehrten Flächen der Klemmkörper zusammenwirkende Nuten aufweisen zur Aufnahme mindestens einer Knochenschraube. Bei einer derartigen Ausbildung können die Knochenschrauben in allen drei Freiheitsgraden verstellt werden. Dies ist für manche Anwendungsfälle von Vorteil.

Zusätzlich oder alternativ kann auf die Außenseite der erfindungsgemäßen Trägerstange eine Haltevorrichtung aufgeschoben werden, die die Trägerstange zumindest teilweise umgreift. Mittels einer Feststellschraube kann sie in ihrer axialen Lage an der Trägerstange festgelegt werden. Die Haltevorrichtung weist eine Querbohrung auf zur Aufnahme eines die Klemmvorrichtung haltenden Schaftes, der seinerseits in der Querbohrung festlegbar ist. Mit Hilfe des Schaftes kann die Klemmvorrichtung in veränderlichem Abstand zur Trägerstange gebracht werden. Die Knochenschrauben können in die Knochenteile an im wesentlichen beliebige Positionen gesetzt werden und durch die erfindungsgemäße Vorrichtung aufgrund der vielen Freiheitsgrade sicher gehaltert werden. Nach einer Ausgestaltung der zuletzt erwähnten Ausführungsform ist vorgesehen, daß die Haltevorrichtung aus je zwei C-förmigen Backenhälften besteht, deren Schenkel verschieden ausgebildet sind, wobei die Backenhälften um die Trägerstange legbar und durch eine Stellschraube derart gegeneinander verspannbar sind, daß bei angezogener Stellschraube die Haltebacke die Trägerstange umfaßt und durch diagonale Verspannung auf dieser fixiert wird. Die diagonale Verspannung hat den Vorteil einer besonders sicheren Fixierung der Halterung auf der Trägerstange, da die Halterung alle vier Flächen der Trägerstange gleichmäßig belastet. Eine derartige Halterung hat den Vorteil, daß sie jederzeit von der Seite auf die Trägerstange montiert und wieder entfernt werden kann.Es ist daher möglich, im Bedarfsfall auch nachträglich an einer beliebigen Stelle der Trägerstange eine zusätzliche Haltebacke anzubringen, um weitere Knochenschrauben sicher zu haltern. Um auch bei einer solchen Konstruktion eine dynamische Halterung der Knochenteile zu ermöglichen, sind die Backenhälften einer derart ausgebildeten Haltebacke durch eine Halteschraube derart miteinander verschraubbar, daß in gelöstem Zustand der Halteschraube die Haltebacke auf der Trägerstange frei verschiebbar,aber gegen Verdrehen und Kippen gesichert ist.

Der in einer derartigen Halterung festlegbare Schaft trägt seinerseits eine Klemmvorrichtung, in der die Knochenschraube in ihrer axialen Position festgelegt werden kann.

Sie kann aus zwei Klemmbacken bestehen, die wahlweise um die Schaftachse drehbar sind und mit Hilfe z.B. einer Klemmschraube gegeneinander gespannt werden kann. Die schaftnahe Klemmbacke können jedoch auch über ein Gelenk gegenüber dem Schaft verschwenkbar sein, um eine größere Bewegungsfreiheit für die Knochenschraube zu ermöglichen.

Wie aus der voranstehenden Beschreibung deutlich wird, ist der erfindungsgemäße Fixateur außerordentlich vielseitig und variabel einsetzbar, z.B. als System mit einer einzigen starren Trägerstange oder als ein aus mehreren Trägerstangen bestehendes System, bei dem die Trägerstangen über gelenkige, jedoch feststellbare Verbindungsmittel unter beliebigen Winkeln miteinander verbindbar sind. In allen Fällen, auch bei einer einzigen starren Trägerstange ist eine völlige variable Einrichtung der Knochenfragmente beim angebrachten, aber noch nicht endgültig fixierten System möglich.

Eine passive Dynamisierung mittels eines Antriebs über die in der Trägerstange sitzenden Gleitstücke benötigt nur einen mittigen Kraftangriffspunkt. Dies ist im Gegensatz zu bekannten Vorrichtungen nur bei der Erfindung möglich.

Die Erfindung wird nachfolgend anhand von Zeichnungen näher erläutert.

Fig. 1 zeigt eine perspektivische und vereinfachte Darstellung einer am Knochenteil befestigten ersten Ausführungsform nach der Erfindung.

Fig. 2 zeigt einen Schnitt entlang der Linie II-II nach Fig. 1.

Fig. 3 zeigt eine Ansicht der Vorrichtung nach Fig. 2 von einer Seite.

Fig. 4 zeigt eine Ansicht einer Klemmbacke ohne Knochenschraube mit über ein Kippgelenk angelenktem Schaft.

Fig. 5 zeigt eine Draufsicht auf die Klemmbacke nach Fig.4.

Fig. 6 zeigt eine Endansicht der Klemmbacke nach Fig. 4.

Fig. 7 und 8 zeigen eine Seitenansicht bzw. eine Endansicht einer Backenhälfte der Haltebacke.

Fig. 9 und 10 zeigen eine Seitenansicht bzw. eine Endansicht einer anderen Backenhälfte der Haltebacke.

Fig. 11 zeigt eine perspektivische und vereinfachte Darstellung einer an Knochenteilen befestigten zweiten Ausführungsform einer Vorrichtung nach der Erfindung.

Fig. 12 zeigt einen Ausschnitt in Draufsicht auf die Vorrichtung nach Fig. 11.

Fig. 13 zeigt einen Schnitt entlang der Linie X-X nach Fig. 11.

Fig. 14 zeigt eine Draufsicht auf ein Gleitstück der Ausführungsform nach Fig. 11.

Fig. 15 zeigt eine Seitenansicht des Gleitstücks nach Fig. 14.

Fig. 16 zeigt eine Draufsicht auf eine dritte Ausführungsform der Vorrichtung nach der Erfindung.

Fig. 17 zeigt eine Seitenansicht der Vorrichtung nach Fig. 16 mit eingesetzten Knochenschrauben.

Fig. 18 zeigt einen Querschnitt durch eine der beiden Trägerstangen.

Fig. 19 zeigt mehrere zusammengesetzte Trägerstangen einer Vorrichtung nach den Figuren 16 bis 18.

Fig. 20 zeigt perspektivisch eine erste Ausführungsform eines Gleitstücks.

Fig. 21 zeigt perspektivisch ein Druckelement für das Gleitstück nach Fig. 20.

Fig. 22 zeigt perspektivisch eine Lagerkugel für ein Gleitstück.

Fig. 23 zeigt die Draufsicht auf eine Ringfeder für eine Lagerkugel nach Fig. 22.

Fig. 24 zeigt perspektivisch eine andere Ausführungsform eines Gleitstücks für die Vorrichtung nach den Figuren 16 bis 18.

Fig. 25 zeigt perspektivisch ein Anschlußstück für eine Trägerstange nach den Figuren 16 bis 19.

Fig. 26 zeigt perspektivisch ein modifiziertes Anschlußstück mit Schaft.

Fig. 27 zeigt perspektivisch ein anderes Verbindungsteil.

Fig. 28 zeigt perspektivisch eine Ausführungsform für eine Klemmvorrichtung mit Gelenkkugel.

Fig. 29 zeigt die Draufsicht auf eine Knochenschraube für eine in den voranstehenden Figuren dargestellte Vorrichtung.

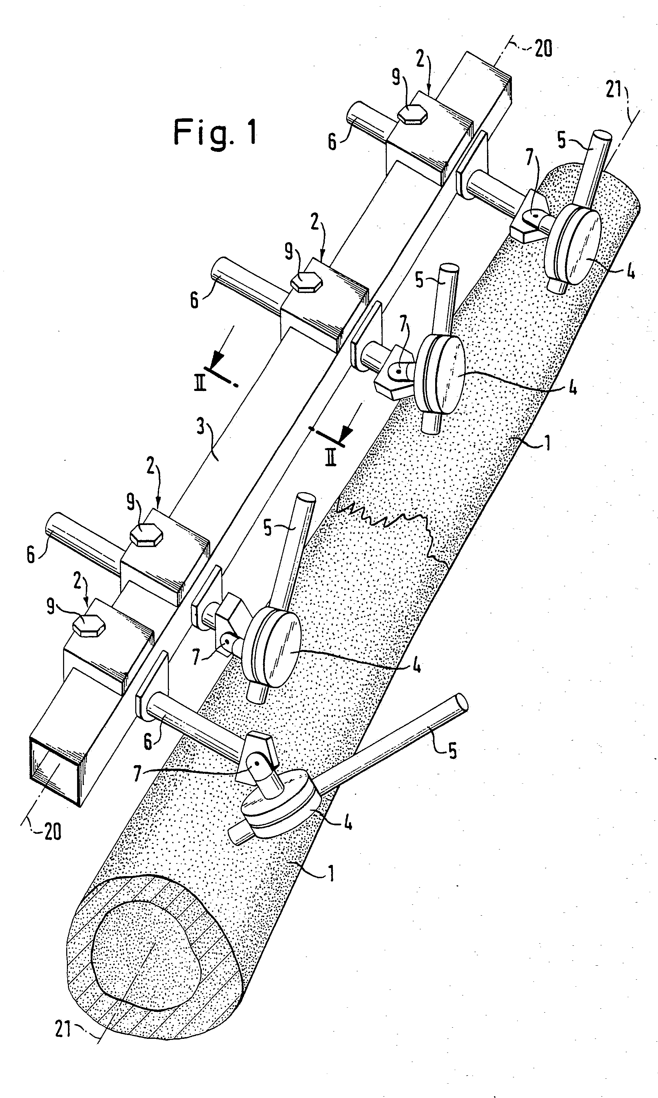

Bei der Gesamtdarstellung der ersten Ausführungsform nach Fig. 1 sind zwei Knochenteile 1 schematisch angedeutet, die zum Zwecke der Heilung in eine genau definierte räumliche Position zueinander gebracht werden sollen. An einer Trägerstange 3, die ein quadratisches Profil hat und vorzugsweise hohl ausgebildet ist, sind mehrere Haltebacken 2 befestigt und können nach Lösen einer Stellschraube 9 in Längsrichtung der Trägerstange 3 verschoben und anschließend durch Anziehen wieder fixiert werden. An jeder Haltebacke 2 ist mittels eines in dieser quer verschiebbaren Schaftes 6 eine Klemmbackenanordnung 4 gehaltert, die außerdem über ein Kipgelenk 7 gekippt werden kann. An jeder Klemmbackenanordnung 4 sind eine oder zwei Knochenschrauben 5 drehbar gehaltert, die in den entsprechenden Positionen in die Knochenteile 1 eingeschraubt sind.

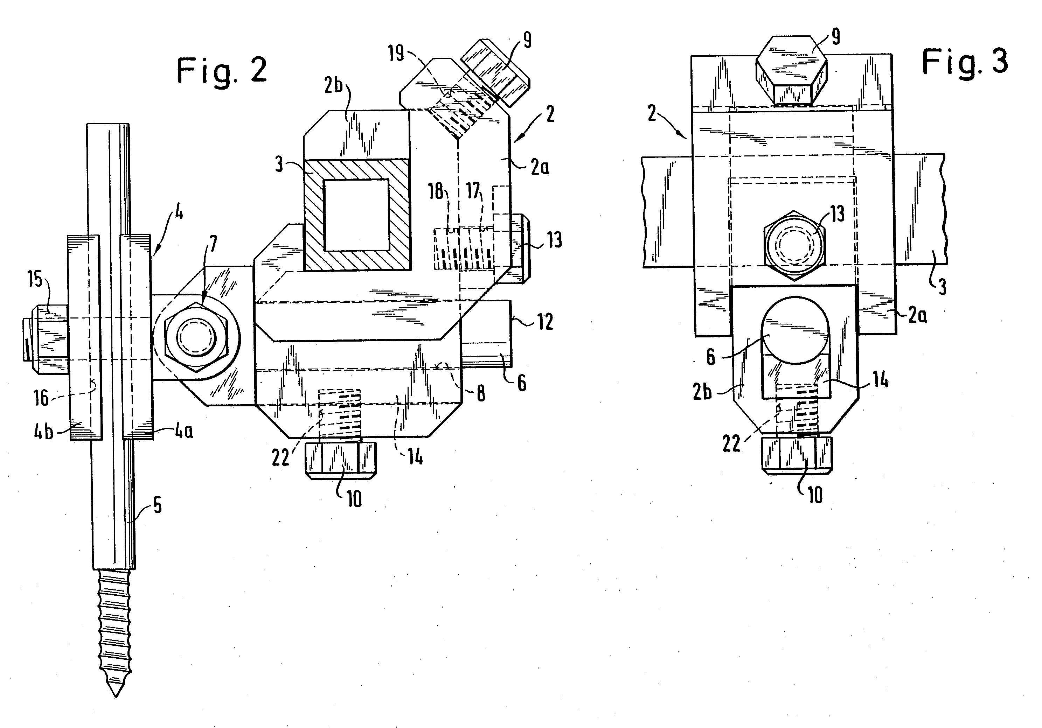

Aus den Fig. 2 bis 10 gehen nun weitere Einzelheiten der ersten Ausführungsform der erfindungsgemäßen Vorrichtung hervor. Es ist zu sehen, daß innerhalb einer Querbohrung 8 jeder Haltebacke 2 der Schaft 6 in Querrichtung verschiebbar gelagert ist, so daß der seitliche Abstand der Knochenschraube 5 gegenüber der Trägerstange in weiten Grenzen verstellt werden kann. Darüber hinaus kann der Schaft 6 mit der Klemmbackenanordnung 4 gegenüber der Haltebacke 2 verdreht werden. Um eine stabile Fixierung des Schaftes 6 in Drehrichtung gegenüber der Haltebacke 2 zu ermöglichen, ist der Umfang des Schaftes 6 vorzugsweise mit einer in Längsrichtung verlaufenden Feinprofilierung versehen, und eine Fixierung des Schaftes 6 gegenüber der Haltebacke 2 erfolgt durch Anziehen einer Stellschraube 10, die in eine Gewindebohrung 22 in der Haltebacke 2 einschraubbar ist und über ein Klemmstück 14 - das ebenfalls profiliert ist - radial gegen den profilierten Schaft 6 drückt. Es ist jedoch auch möglich, anstelle der Profilierung sowohl den Außenumfang des Schaftes 6 als auch die Querbohrung 8 mit einem verdrehungssicheren Profil, z.B. einer Feinverzahnung zu versehen.

Zwischen dem Schaft 6 und der Klemmbackenanordnung 4 ist ein Kippgelenk 7 vorgesehen, dessen Kippachse in der Normalstellung des Schaftes 6 gegenüber der Haltebacke 2 parallel zur Längsrichtung der Trägerstange 3 verläuft. Durch dieses Kippgelenk 7 ist es möglich, die Klemmbackenanordnung 4 mit der Kno-chenschraube 5 in eine Schräglage zu kippen, um ein seitliches Einschrauben der Knochenschraube 5 in die Knochenteile 1 zu ermöglichen.

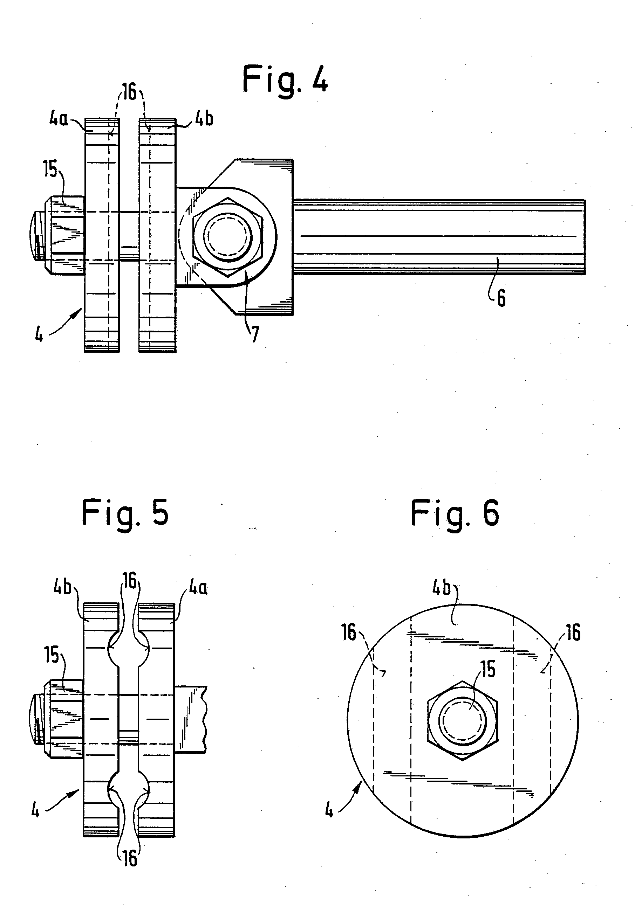

Schließlich ist die Klemmbackenanordnung 4 derart ausgeführt, daß die die Knochenschraube 5 aufnehmende Halterung, die mit Aufnahmerinnen 16 zwischen den zwei Klemmbacken 4a, 4b (Fig. 5) geformt ist, um die Achse einer Verschraubung 15 gedreht und anschließend fixiert werden kann.

Am freien Ende 12 des Schaftes 6 sind vorzugsweise zusätzliche Befestigungsmittel vorgesehen (nicht gezeigt), um eine weitere Klemmbackenanordnung 4 auch auf dieser Seite zu befestigen. Diese Befestigungsmittel können z.B. in Form einer Gewindebohrung oder eines Gewindeschaftes ausgebildet sein.

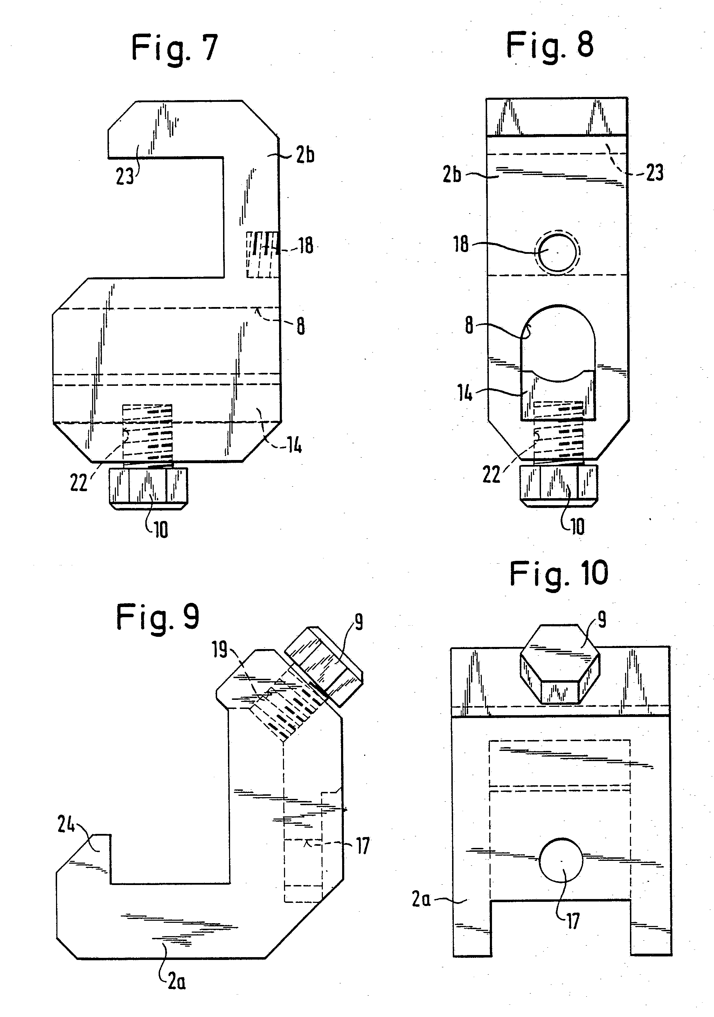

Schließlich ist insbesondere in den Fig. 7 bis 10 zu sehen, daß die Haltebacke 2 aus zwei Teilen 2a und 2b zusammengesetzt ist, so daß ein seitliches Aufsetzen auf die Trägerstange 3 möglich ist. Hierzu weisen die Backenteile 2a und 2b eine C-Form mit verschiedenen Längen ihrer Schenkel 23, 24 auf. Die Backenhälfte 2a (Fig. 9, 10) läßt sich (in der Zeichnung nach Fig. 2 gesehen) von unten her auf die Trägerstange 3 aufsetzen, während die Backenhälfte 2b (Fig. 7, 8) von der rechten Seite aus (in der Zeichnung nach Fig. 2 gesehen) auf die Trägerstange 3 aufgeschoben werden kann. Beide Backenhälften greifen dabei ineinander, wie insbesondere aus Fig. 3 ersichtlich ist. Zur Fixierung einer in dieser Weise auf die Trägerstange 3 geschobenen Backe 2 wird eine Stellschraube 9 in eine Gewindebohrung 19 eingeschraubt, die die beiden Backenhälften 2a und 2b derart ineinanderdrückt, daß diese die Trägerstange 3 fest und formschlüssig umgreifen. Beim vollständigen Anziehen der Stellschraube 9 werden die beiden Backenteile diagonal so gegeneinander verspannt, daß sie reibungsschlüssig auf der Trägerstange 3 fixiert werden.

Um im Laufe des Heilungsprozesses der Knochenteile 1 auch eine dynamische Fixierung zu ermöglichen, kann nach Lösen der Stellschraube 9 eine weitere Halteschraube 13 durch eine Bohrung 17 in der Backenhälfte 2a in eine Gewindebohrung 18 in der Backenhälfte 2b eingeschraubt werden, die die beiden Backenhälften 2a und 2b zwar fest miteinander verbindet, so daß diese die Trägerstange 3 formschlüssig umfassen, eine Verspannung der beiden Backenhälften gegeneinander jedoch nicht erfolgt. Hierdurch kann eine derart verschraubte Haltebacke 2 frei auf der Trägerstange 3 in Längsrichtung gleiten, ist jedoch gegen Verdrehen und Kippen einwandfrei gesichert.

Als Halteschraube 13 kann die herausgeschraubte Stellschraube 9 verwendet werden, die bei der dynamischen Halterung nicht angezogen werden darf.

Durch seitliche Verstellbarkeit der Klemmbacken 4 bzw. der Knochenschrauben 5 gegenüber der Trägerstange 3 ist es möglich, die Längsachse 20 der Trägerstange 3 gegenüber der Längsachse 21 der Knochenteile 1 (siehe Fig. 1) genau parallel auszurichten, was insbesondere bei der dynamischen Fixierung der Knochenteile, also nach Lösen der Längsfixierung der Haltebacken 2 von großem Vorteil ist, da dann keine seitlichen Kräfte auf die Fraktur ausgeübt werden.

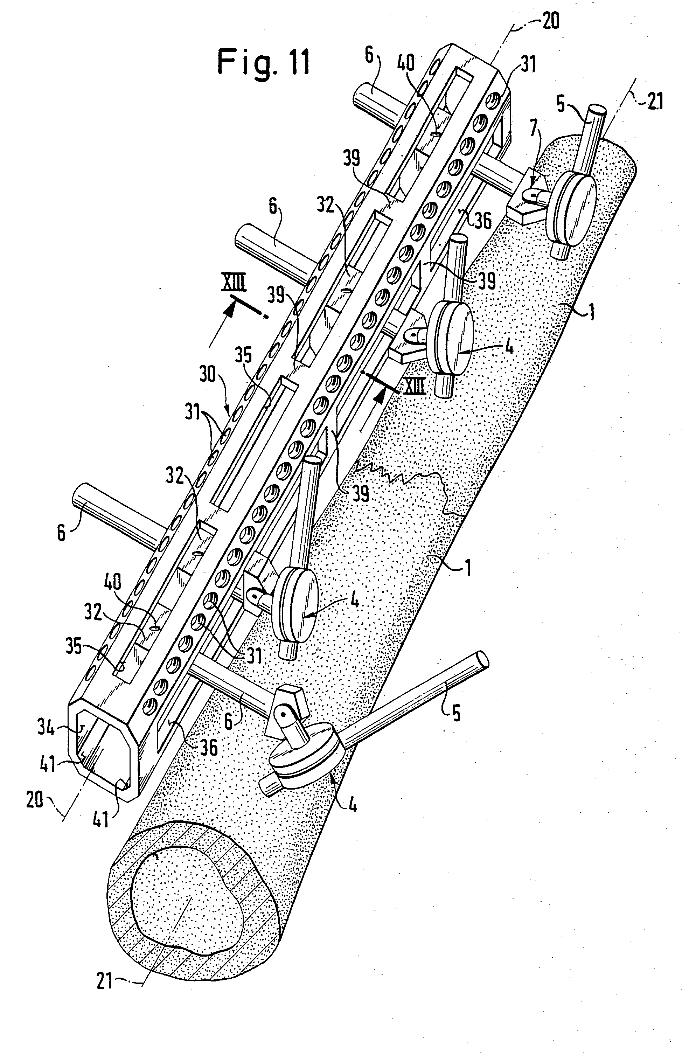

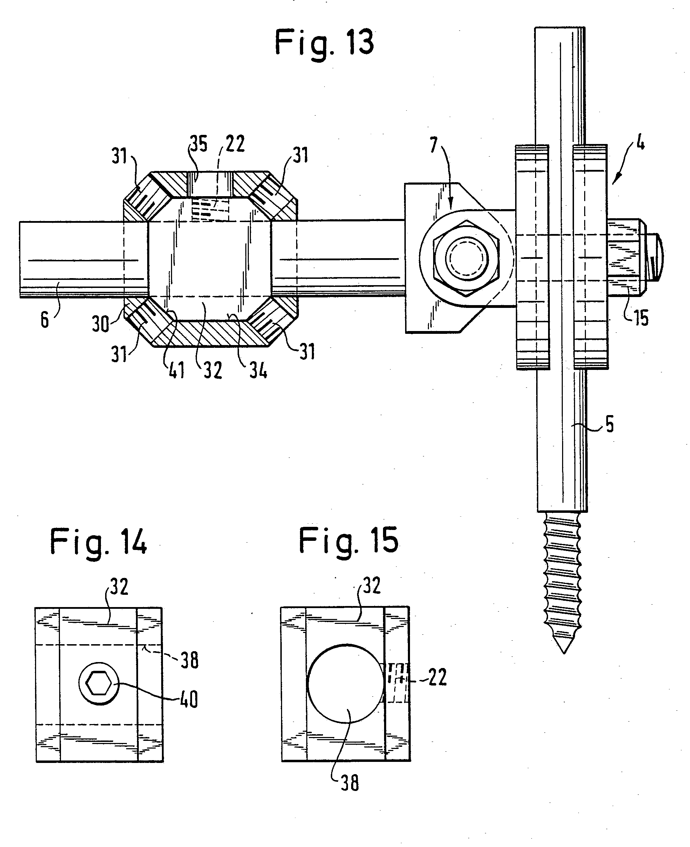

Nachfolgend soll nun eine zweite Auführungsform der erfindungsgemäßen Vorrichtung in Verbindung mit den Fig. 11 bis 15 beschrieben werden. Die Fig. 11 ähnelt der perspektivischen Darstellung nach Fig. 1; im Gegensatz zu der ersten Ausführungsform ist bei der zweiten Ausbildung der Erfindung jedoch eine geschlossene Bauweise für die Vorrichtung gewählt. Eine Trägerstange 30, die hohl ausgebildet ist, weist ein verdrehungssicher ausgebildetes Innenprofil in Form einer Längsaussparung 34 auf, in der Gleit stücke 32 verdrehungssicher und innen längsverschieblich geführt sind (siehe insbesondere Fig. 13) Das verdrehungssichere Profil der Längsaussparung 34 in der Trägerstange 30 und die Außenkontur der Gleitstücke 32 sind in der Form eines quadratischen Profils ausgebildet, an denen die Eckbereiche 41 abgeschrägt sind. Es wird also auch die Form eines unregelmäßigen Achtecks gebildet.

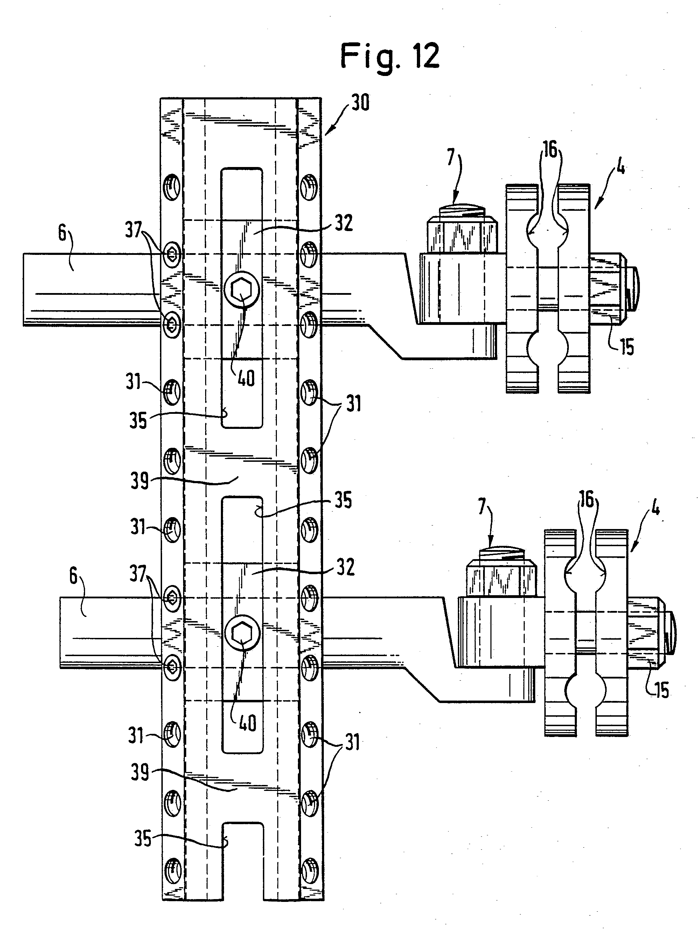

Eine Fixierung der Gleitstücke 32 in Längsrichtung der Trägerstange 30 ist dadurch möglich, daß in den außen abgeschrägten Eckbereichen 41 der Trägerstange 30 diagonal angeordnete Gewindebohrungen 31 vorgesehen sind, in die Stellschrauben 37 (siehe Fig. 12) derart einschraubbar sind, daß die Gleitstücke 32 fest gegen die gegenüber angeordneten Eckbereiche 41 der Trägerstange 30 gedrückt werden. Die Stellschrauben 37 sind vorzugsweise als Imbus- oder Madenschrauben ausgebildet.

In einer entsprechenden Querbohrung 38 (Fig. 14 und 15) der Gleitstücke 32 ist der Schaft 6 (ähnlich dem ersten Ausführungsbeispiel in Querrichtung verschiebbar angeordnet und kann durch eine in eine Gewindebohrung 22 einschraubbare Stellschraube 40, vorzugsweise einer Inbusschraube, fixiert werden. Der Schaft 6 ist zu beiden Seiten der Trägerstange 30 aus Längsschlitzen 36 (siehe Fig. 11) herausgeführt. Die Stellschraube 40 ist über einen weiteren Schlitz 35 in der Oberseite der Trägerstange 30 zugänglich. Die Schlitze 35 bzw. 36 sind in Längsrichtung durch Querstege 39 unterteilt, um einen Zusammenhalt der Trägerstange 30 zu gewährleisten bzw. die Stabilität zu erhöhen.

Insbesondere aus den Fig. 11 und 12 geht hervor, daß die Gewindebohrungen 31 in den Eckbereichen 41 der Trägerstange 30 in Form von vier Reihen angeordnet sind, um die Gleitstücke 32 an praktisch jeder Längsposition der Trägerstange 30 fixieren zu können. Zur Fixierung jedes Gleitstücks 32 werden vorzugsweise zwei Stellschrauben 37 in die entsprechenden Gewindebohrungen 31 eingeschraubt.

Die zweite Ausführungsform nach den Fig. 11 bis 15 hat gegenüber der ersten Ausführungsform nach den Fig. 1 bis 10 den Vorteil, daß durch die geschlossene Bauweise sich verminderte Abstände zwischen den Knochenschrauben 5 und der Trägerstange 30 ergeben. Dies führt nicht nur zu einer besseren Stabilität der Vorrichtung, sondern erleichtert auch das Tragen durch den Patienten. Es ist möglich, die zweite Ausführungsform nicht nur mit querverschiebbaren Klemmbacken 4 auszurüsten, sondern die Knochenschrauben können näher an die Gleitstücke 32 heran montiert werden, wenn für einige Klemmvorrichtungen auf die Querverschieb lichkeit verzichtet wird. So ist es z.B. möglich,die Gleitstücke 32 mit einer Halterung zu versehen, in der die Knochenschrauben 5 direkt befestigt und über entsprechende Aussparungen oder Schlitze am Boden der Trägerstange 30 nach unten herausgeführt werden.

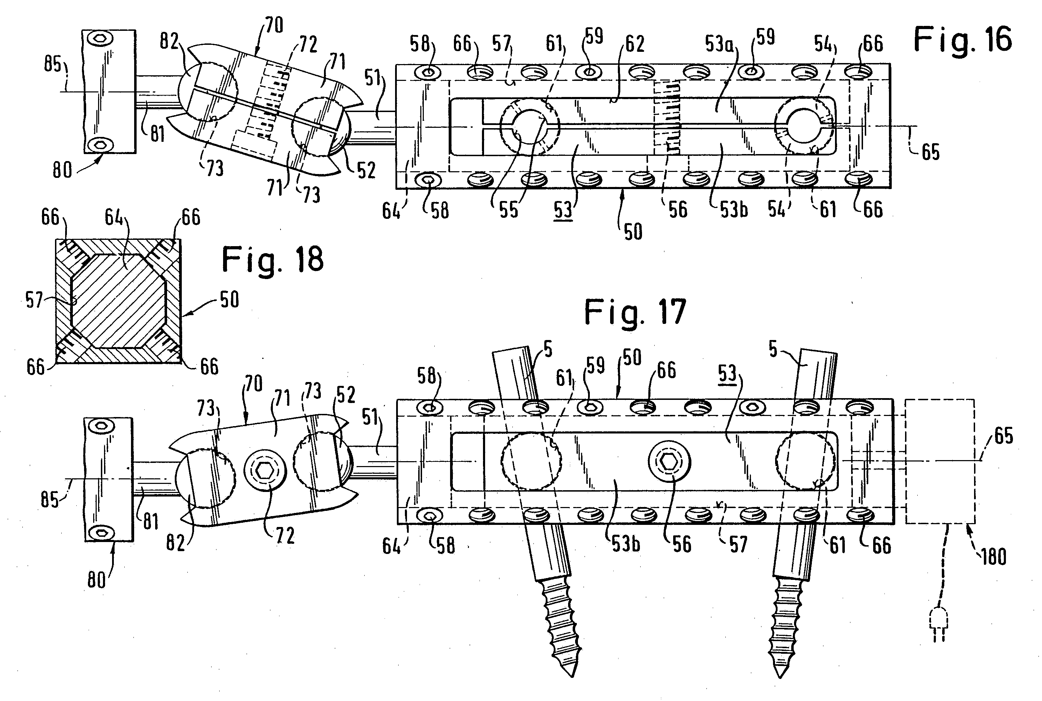

Die in den Fig. 16 und 17 dargestellte Vorrichtung zum Fixieren von Knochenteilen gegeneinander besteht aus zwei Trägerstangen 50 und 80, die durch ein Verbindungsglied 70 in Längsrichtung aneinander gekoppelt sind. Von der (in der Zeichnung) links dargestellten Trägerstange 80 ist nur das Ende gezeigt; sie ist wie die rechts dargestellte Trägerstange 50 aufgebaut. Die beiden Trägerstangen 50 und 80 weisen Längsachsen 65 bzw. 85 auf und können mittels des Verbindungsgliedes 70 gegeneinander verdreht, gekippt und versetzt werden, um die gleichen Freiheitsgrade der an den Trägerstangen 50 bzw. 80 befestigten Knochenteile (nicht gezeigt) zu ermöglichen.

Die beiden Trägerstangen 50 und 80 sind hohl ausgebildet und weisen ein verdrehungssicher ausgebildetes Innenprofil in Form einer Längsaussparung 57 auf. In dieser Längsaussparung 57 ist ein Gleitstück 53 für Knochenschrauben 5 verdrehungssicher und längsverschieblich geführt, wobei das Gleitstück 53 zwei Gleitstücke 53a, 53b aufweist. Die verdrehungssichere Führung erfolgt auf die Weise, daß das Außenprofil des Gleitstücks 53 dem Innenprofil 57 angepaßt ist. Die Außenkontur des Gleitstücks 53 und das Innenprofil 57 sind im vorliegenden Fall in der Form eines quadratischen Profils ausgebildet, in denen die Eckbereiche abgeschrägt sind. Es wird also die Form eines unregelmäßigen Achtecks gebildet.

Das Gleitstück 53 weist zwei Kugelpfannen 61 auf, in denen Kugelhalbschalen 54 angeordnet sind. Die Kugelhalbschalen 54 weisen im Inneren je eine Bohrung 55 auf, in die Knochenschrauben 5 (siehe Fig. 17) eingesteckt werden können. Die Gleitstücke 53a, 53b können durch eine Fixierschraube 56 gegeneinander und auf die Halbschalen 54 gepreßt werden, wodurch die Knochenschrauben 5 in ihrer jeweils eingestellten Position gegenüber dem Gleitstück 53 fixiert werden. Aufgrund der in den Kugelpfannen 61 angeordneten Halbschalen 54 können die Knochenschrauben 5 um einen berenzten Winkel in allen Richtungen gekippt werden, um ein schräges Einsetzen in die Knochenteile zu ermöglichen.

Die durch die Fixierschraube 56 miteinander verspannten Haltebacken 53a,53b der Gleitstücke 53 können innerhalb des Innenprofils 57 der Trägerstange 50 (bzw. der Trägerstange 80) in Richtung der Längsachse 65 verschoben werden, um eine genaue Einstellung vorzunehmen oder eine dynamische Halterung zu ermöglichen.Eine Fixierung der Gleitstücke 53 gegenüber der Trägerstange 50 erfolgt durch Anziehen von Klemmschrauben 59, die in Gewindebohrungen 66 (siehe auch Fig. 18) eingeschraubt werden und auf die Eckbereiche der Gleitstücke 53 drücken.Es versteht sich, daß die Kanten der Trägerstangen 50, 80 ebenfalls gefast sein können wie z.B. in Fig. 11 gezeigt.

Die Trägerstangen 50 und 80 sind an den Seitenflächen mit Längsschlitzen 62 und 63 versehen, aus denen zum einen die Knochenschrauben 5 herausgeführt sind und über die zum anderen die Fixierschraube 56 zugänglich ist. In den Endbereichen sind die Trägerstangen 50 bzw. 80 ungeschlitzt, und bei größerer Länge sind die Schlitze 62, 63 unter Umständen aus Stabilitätsgünden durch Zwischenstege (nicht gezeigt) überbrückt.

Die Verbindung zwischen den Trägerstangen 50 und 80 und dem Verbindungsglied 70 erfolgt durch Verbindungsstäbe 51 bzw. 81, die mittels eines Ansatzstückes 64 im Innenprofil 57 befestigt werden. Die Befestigung erfolgt durch Feststellschrauben 58, die in Gewindebohrungen 66 im Endbereich (siehe auch Fig. 18) eingeschraubt werden. Wie Fig. 18 erkennen läßt, ist das Außenprofil des Ansatzstückes 64 dem Innenprofil 57 der Trägerstange 50 angepaßt. Die Feststellschrauben 58 (siehe Fig. 1 und 2) sind in Fig. 3 jedoch nicht gezeigt.

An den freien Enden der Verbindungsstäbe 51 bzw. 81 sind Kugeln 52 bzw. 82 angeordnet. Das Verbindungsglied 70 besteht aus zwei Hälften 71, die mittels einer Klemmschraube 72 gegeneinander gepreßt werden. Die beiden Hälften 71 sind mit zwei im Abstand voneinander angeordneten Kugelpfannen 73 versehen, die die Kugeln 52 bzw. 82 an den Verbindungsstäben 51 bzw. 81 umschließen und mit diesen zusammen zwei hintereinander angeordnete Kugelgelenke bilden.

Die Fig. 16 und 17 zeigen, daß durch das verhältnismäßig kurz ausgebildete Verbindungsglied ein Verdrehen, ein Verschwenken bzw. Knicken und ein Versetzen der Trägerstangen 50 und 80 gegeneinander möglich ist.Hierdurch können die bereits an den Trägerstangen 50 bzw. 80 befestigten Knochenteile noch gegeneinander verdreht, gekippt oder versetzt werden, ohne daß hierzu das Gleitstück 53 für die Knochenschrauben 5 gelöst werden muß. Eine Verschiebung der beiden Trägerstangen 50, 80 und damit der daran befestigten beiden Knochenteile gegeneinander genau in Richtung der Längsachsen 65 bzw. 85 kann auf einfache Weise durch Lösen der Klemmschraube 59 erfolgen. Dieses Verschieben kann entweder für Korrekturzwecke oder zwecks dynamischer Fixierung vorgenommen werden.

Im vorliegenden Ausführungsbeispiel weist jede Trägerstange 50 bzw. 80 ein zweiteiliges Gleitstück 53 mit je zwei Knochenschrauben 5 auf. Es ist selbstverständlich auch möglich, mehr als zwei Knochenschrauben 5 vorzusehen, oder innerhalb einer Trägerstange 50 mehr als ein solches Gleitstück 53 verschiebbar zu lagern. Schließlich ist es auch möglich, innerhalb der Trägerstange 50 bzw. 80 eine einfacher aufgebaute Halterung vorzusehen, die lediglich eine oder mehrere Durchgangsbohrungen zur nicht-kippbaren Halterung einer Knochenschraube 5 aufweist, falls ein Kippen der Knochenschrauben 5 in Sonderfällen nicht notwendig ist. Eine solche Halterung (in den Zeichnungen nicht gezeigt) kann anstelle des normalen Gleitstücks 53 in die Trägerstange eingesetzt werden und bedeutet einen geringeren Aufwand sowie ein einfacheres Einstellen.

Auch wenn die vorliegende Erfindung in Verbindung mit zwei Trägerstangen 50 und 80 beschrieben wurde, die durch ein Verbindungsglied 70 einstellbar miteinander gekoppelt sind, so ist es selbstverständlich auch möglich, mehr als zwei Trägerstangen durch entsprechende Verbindungsglieder miteinander zu koppeln. So ist es auf einfache Weise möglich, auch in das (in der Zeichnung) rechte Ende der Trägerstange 50 mittels eines Verbindungsstabes ein weiteres Verbindungsglied zu befestigen und somit eine weitere Trägerstange anzukoppeln, wenn z.B. drei Knochenteile gegeneinander fixiert werden sollen.

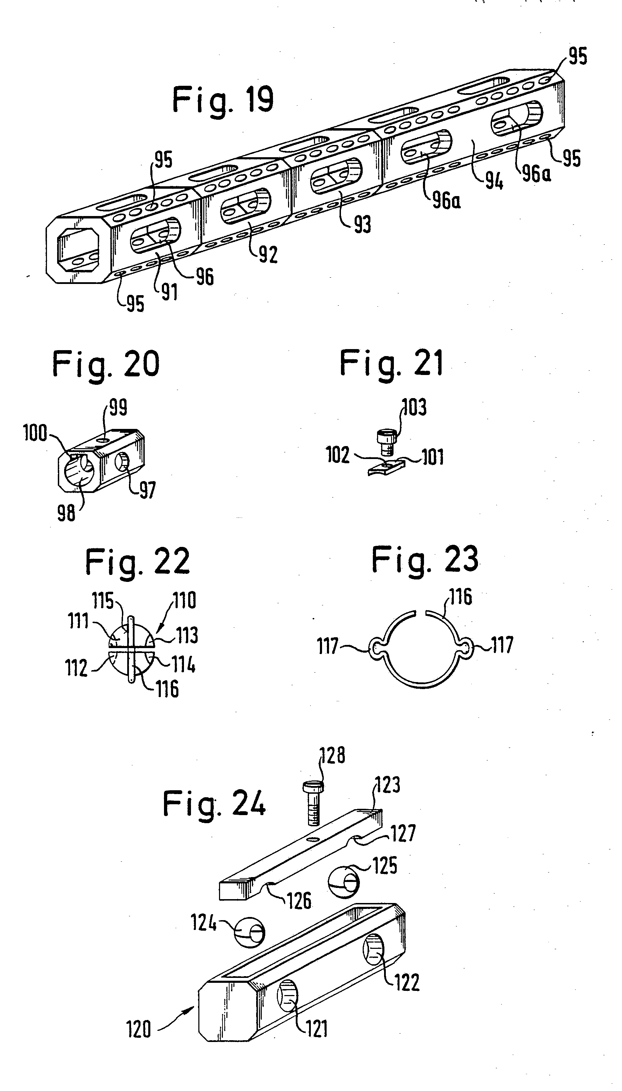

In Fig. 19 sind mehrere Trägerstangen 91, 92, 93 und 94 gezeigt, die in ihrem Aufbau den Trägerstangen 50 nach den Fig. 16 und 17 gleichen. Es ist jedoch zu erkennen, daß sie auch an den Außenkanten angefast sind zur Aufnahme einer Reihe von Gewindebohrungen 95 an jeder der gefasten Seitenkanten. Die Trägerrohre 91 bis 93 haben Einheitsmaß, z.B. eine Länge von 85 mm. Das Trägerrohr 94 hat ebenfalls Einheitsmaß und eine Länge von z.B. der doppelten Länge, also 170 mm. Die kleineren Trägerrohre 91, 92, 93 haben in jeder Seitenfläche einen länglichen ovalen Schlitz 96, während das längere Trägerrohr 94 zwei ovale Schlitze 95 aufweist. Die Trägerrohre 91 bis 94 sind über nicht gezeigte Kupplungsstücke starr miteinander verbunden. Die Kupplungsstücke sind relativ kurze Elemente, beispielsweise von einer Länge von 20 mm, deren Außenkontur der Innenkontur der Trägerrohre entspricht. Sie werden mit den Enden der Trägerrohre über die Feststellschrauben mit diesen verbunden, die in die entsprechenden Bohrungslöcher 95 eingeschraubt werden. Auf diese Weise kann eine beliebige Länge eines Trägerrohrs je nach Versorgungsfall hergestellt werden.

Fig. 20 zeigt ein Gleitstück, das passend in eines der Trägerrohre 91 bis 94 eingeschoben und mit Hilfe von mehreren, bereits erwähnten, jedoch nicht gezeigten Feststellschrauben in einer beliebigen axialen Position im Trägerrohr festgesetzt werden kann. Es hat zum Beispiel eine Länge von 15 mm und ist von einer Querdurchbohrung 97 und einer Längsdurchbohrung 98 durchsetzt. Außerdem weist eine weitere Fläche eine Gewindebohrung 99 auf. In der der Bohrung 99 zugekehrten Seite der Wandung der Längsdurchbohrung 98 ist eine Nut 100 geformt. Sie dient zur Aufnahme eines in Fig. 21 gezeigten Druckelements 101, das z.B. eine Ausnehmung 102 aufweist zur Aufnahme einer in die Gewindebohrung 99 eingeschraubten Gewindeschraube 103. Mit Hilfe der Schraube 103 wird das Druckelement 101 in der Nut 100 gehalten und gegen eine durch die Querbohrung 97 hindurchgeführte Knochenschraube, wie sie voranstehend bereits beschrieben wurde, angepreßt, um diese in gewünschter axialer Lage in dem zugeordneten Trägerrohr zu fixieren, wobei die Knochenschraube über einen Schlitz 96 des Trägerrohrs nach außen steht. Es versteht sich, daß die konkave Unterseite des Druckelements so in der Nut 100 sitzt, daß eine Anpassung an die Rundung der Knochenschraube erfolgt.

In das Gleitstück nach Fig. 20 kann jedoch auch eine geteilte Lagerkugel 110 nach Fig. 22 eingesetzt werden, und zwar über die Längsdurchbohrung 98, wobei im Inneren der Längsdurchbohrung in Höhe der Querdurchbohrung 97 kugelpfannenartige Ausnehmungen vorgesehen sind. Die Kugelschalen 111, 112 weisen jeweils eine halbzylindrische Ausnehmung 113 bzw. 114 auf, die zusammen eine Durchbohrung zur Aufnahme einer Knochenschraube bilden. Werden die Kugelschalen 111, 112 zusammengepreßt, beispielsweise mit Hilfe des Druckelements 101 nach Fig. 21, wird die aufgenommene Knochenschraube axial festgelegt. Außerdem wird die Kugel ihrerseits in ihrer Drehlage fixiert. Das Druckstück 101 ist für diesen Fall mit einer kugeligen Ausnehmung verbunden, die mit der zugeordneten Kugelschale in Eingriff tritt, um eine unterschiedliche Drehlage der Kugel zu gestatten. Die Kugelschalen 111, 112 weisen auch eine Ringnut 115 auf zur Aufnahme einer Ringfeder 116, wie in Fig. 23 dargestellt. Die z.B. aus Federdraht bestehende Ringfeder ist geteilt und weist diametral gegenüberliegend Ausbuchtungen 117 auf. Die Ringfeder 116 wird in die Nuten 115 eingelegt, sie ist jedoch so bemessen, daß die Feder im entspannten Zustand einen geringen Abstand der Kugelschalen 111, 112 bewirkt. Mit Hilfe der Ringfeder 116 sind daher die Kugelschalen miteinander verbunden. Es sei noch bemerkt, daß die Nuten 115 sich in einer Ebene befinden, die senkrecht zur Achse der Durchbohrung verläuft. Das Gleitstück nach Fig. 20 weist im unteren Bereich eine Aussparung auf (nicht gezeigt), der auch eine Aussparung im Druckstück 101 entspricht, wobei in diesen Aussparungen die Ausbuchtungen 117 der Ringfeder 116 aufgenommen werden. Sie sind so bemessen, daß die Kugelschalen 111, 112 zwar an einem Herausfallen aus dem Gleitstück gehindert werden, die Kugel indessen eine begrenzte Schwenkbewegung nach allen Richtungen durchführen kann, um eine unterschiedliche Winkellage der Knochenschraube zu ermöglichen.

In Fig. 24 ist ein Gleitstück 120 dargestellt, das den gleichen Querschnitt hat wie das Gleitstück nach Fig. 20. Es besitzt zwei Querdurchbohrungen 121, 122 mit einem Durchmesser, der erheblich größer ist als der Durchmesser der aufzunehmenden Knochenschraube. Dies trifft im übrigen auch auf die Querdurchbohrung 97 für das Gleitstück nach Fig. 20 zu. Das Gleitstück 120 ist als nach oben offener Kasten geformt, dessen offene Seite durch einen Deckel 123 verschließbar ist. Der Deckel ist jedoch in das Innere des Gleitstücks 120 einsetzbar, um Lagerkugeln 124 und 125 in die zugeordnete kugelige Ausnehmung (nicht gezeigt) im Inneren des Gleitstücks 120 anzupressen und in einer gegebenen Drehlage zu fixieren. Zu diesem Zweck hat der Deckel 123 kugelschalenförmige Ausnehmungen 126 bzw. 127. Die Kugeln 124, 125 können wie die nach Fig. 22 aufgebaut sein. Der Deckel 123 wird mit Hilfe einer Feststellschraube 128 ins Innere des Gleitstücks 120 gepreßt.

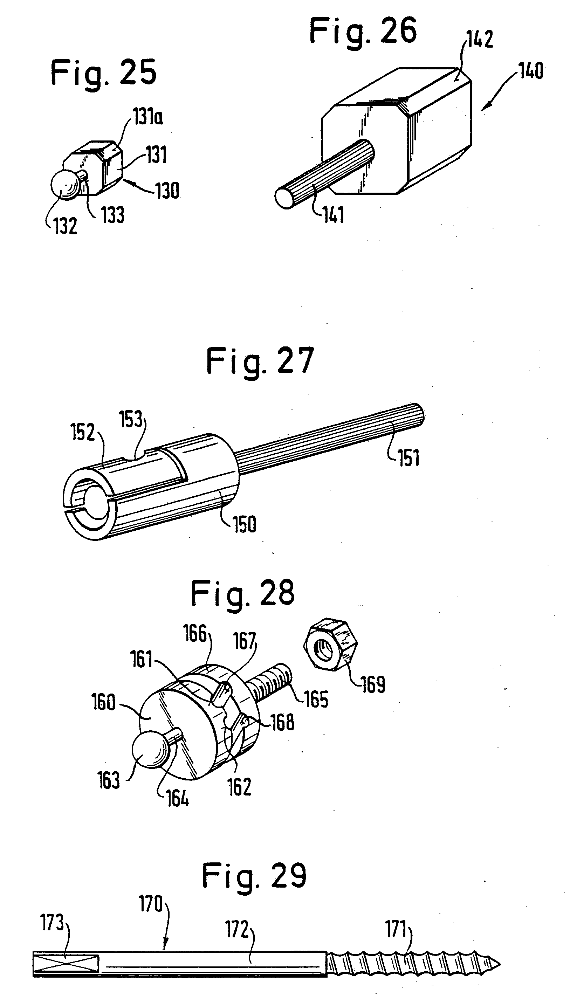

In Fig. 25 ist ein Kugelanschlußstück 130 dargestellt. Es besteht aus einem Klotz 131, dessen Querschnitt dem Querschnitt der Trägerrohre, etwa nach Fig. 19 entspricht. Es wird am Ende eines Trägerrohrs mit Hilfe der bereits beschriebenen Feststellschrauben festgelegt, die z.B. in eine Ansenkung 131a an den gefasten Kanten eingreifen. Der Klotz 131 kann für sich genommen in verlängerter Ausführung auch als Kupplungsstück verwendet werden, um die einzelnen Trägerrohre nach Fig. 19 starr miteinander zu verbinden. Nur nebenbei sei erwähnt, daß auch das kurze Gleitstück nach Fig. 20 diesem Zweck dienen kann. Im vorliegenden Fall ist mit einer Stirnseite des Anschlußstücks 130 eine Gelenkkugel 132 über eine Stange 133 verbunden. Die Kugel 132 gleicht zum Beispiel der Kugel 52 der in den Fig. 16 und 17 gezeigten Vorrichtung. Ohne die Kugel 132 kann die Stange 133 als Schaft mit z.B. strukturierter Oberfläche ausgebildet sein, der in die Querbohrung eines Gleitstücks einer weiteren, quer aufgesetzten Trägerstange eingeführt und fixiert werden kann.

Fig. 26 zeigt ein Schaftanschlußstück 140, das in seiner Kontur und seinen Abmessungen dem Klotz 131 nach Fig. 25 gleicht. An einem Stirnende ist ein Schaft 141 angeformt, der eine Längsstrukturierung aufweist. Der Schaft 141 kann in die Querbohrung eines Gleitstücks, z.B. in einer quer aufgesetzten anderen Trägerstange, eingesteckt werden. Das Schaftanschlußstück 140 weist eine Ansenkung 142 auf, vergleichbar der Ansenkung 131a nach Fig. 25.

In Fig. 27 ist ein zylindrischer Körper 150 dargestellt, an dessen einer Stirnseite eine in Längsrichtung geriffelte Stange 151 angebracht ist. Auf der der Stange 151 gegenüberliegenden Seite ist ein Schalenteil 152 abnehmbar ausgebildet. Es ist von einer Durchbohrung 153 durchsetzt, die sich auch durch das andere fest mit dem Zylinderkörper 150 verbundene Schalenteil hindurcherstreckt. Im Inneren ist eine Kugelpfanne ausgeformt zur Aufnahme beispielsweise einer Gelenkkugel 132 nach Fig. 25. Das Teil nach Fig. 27 kann zum Beispiel an ein Ende eines Trägerrohrs über das Kugelgelenk gelenkig angebracht werden. Die Trägerstange 151 dient zur Aufnahme von Anschlußstücken, die zusätzlich zum Fixateur verwendet werden können. Beispielsweise kann an der Stange 151 eine weitere Klemmvorrichtung zur Aufnahme einer Knochenschraube an einer beliebigen axialen Position der Stange befestigt werden.

In Fig.28 weist ein ringzylindrischer Klemmkörper 160 zwei parallele Nuten 161, 162 auf, die im Querschnitt halbkreisförmig sind. Auf der den Nuten 161, 162 gegenüberliegenden Seite ist eine Gelenkkugel 163 über eine Stange 164 angebracht. An der die Nuten 161, 162 aufnehmenden Seite des Klemmkörpers 160 ist ein Gewindeschaft 165 angebracht. Auf den Gewindeschaft ist ein ringförmiger zweiter Klemmkörper 166 aufgeschoben, der komplementäre Nuten 167, 168 aufweist. Die beiden in Fig. 28 etwas auseinandergezogen gezeichneten Klemmkörper 160, 166 können mit Hilfe einer Mutter 169 gegeneinandergepreßt werden, wobei in den Nuten aufgenommene Knochenschrauben fixiert werden.

Nur der Vollständigkeit halber ist in Fig. 29 eine Knochenschraube abgebildet, beispielsweise eine Schanz'sche Schraube 170. Sie besitzt einen vorderen Gewindeabschnitt 171, einen mittleren glatten Schaftabschnitt 172 und am Ende einen Vierkantabschnitt 172, der mit einem entsprechenden Drehwerkzeug in Eingriff gebracht werden kann.

An das Ende einer Trägerstange bzw. eines Trägerrohrs, wie oben dargestellt, kann ein motorischer Antrieb angebracht werden, z.B. in Form eines Elektromotors, eines Hydraulikoder Pneumatikantriebs,um auf das frei verschiebbare Gleitstück eine permanente oder alternierende axiale Kraft aufzubringen. Sie dient dazu, z.B. auch bei gehunfähigen Patienten mit z.B. gebrochenem Unterschenkelknochen eine Wachstumsstimulation durch eine dynamische Belastung zu erzielen.

## Ansprüche

1. Vorrichtung zum Fixieren von Knochenteilen, mit mindestens einer rohrförmigen Trägerstange, bei der mindestens die Innenkontur im Querschnitt unrund ist, mit mindestens einer an das Querschnittsprofil der Trägerstange angepaßten Haltevorrichtung, die längsverschieblich, jedoch drehfest an der Trägerstange geführt und mit mindestens einer Feststellschraube in einer beliebigen Position einer Trägerstange festlegbar ist, und mit mindestens einer an der Haltevorrichtung angebrachten Klemmvorrichtung, in der eine längliche Knochenschraube oder ein -stift axial verschiebbar geführt und in einer gewünschten axialen Position klemmend festlegbar ist, dadurch gekennzeichnet, daß die Haltevorrichtung als an das Innenprofil der Trägerstange (30, 50, 91 bis 94) angepaßtes Gleitstück (32, 53, 120) ausgebildet ist, das in der Trägerstange frei längsverschieblich geführt ist, die Feststellschraube in einer Gewindebohrung (33, 66, 95) der Trägerstange sitzt und die Trägerstange mit Längsschlitzen (35, 36, 63, 96) versehen ist zum Herausführen einer im Gleitstück festklemmbaren Knochenschraube oder eines Schaftes, der seinerseits die Klemmvorrichtung hält.

2. Vorrichtung nach Anspruch 1, dadurch gekennzeichnet, daß die Längsschlitze (35, 36) durch Querstege (39) unterbrochen sind.

3. Vorrichtung nach Anspruch 1 oder 2, dadurch gekennzeichnet, daß das Innenprofil der Trägerstange die Form eines Quadrates mit abgeschrägten Eckbereichen (41) hat und das Gleitstück (32) entsprechend angepaßt ist und daß in mindestens zwei nebeneinanderliegenden Eckbereichen (41) der Trägerstange (30) je eine Reihe von Gewindebohrungen (31) vorgesehen ist, in die Stellschrauben (37) zwecks Fixierung des Gleitstücks (32) in der Trägerstange (30) einschraubbar sind.

4. Vorrichtung nach Anspruch 3, dadurch gekennzeichnet,daß die Schlitze (35, 96) in allen Seiten des Trägerrohrs geformt sind.

5. Vorrichtung nach einem der Ansprüche 1 bis 4, dadurch gekennzeichnet, daß im Gleitstück (Fig. 20) ein Druckelement (101) angeordnet ist, das mittels einer in einer Gewindebohrung (99) des Gleitstücks sitzenden Feststellschraube gegen eine Knochenschraube anpreßbar ist.

6. Vorrichtung nach Anspruch 5, dadurch gekennzeichnet, daß das Gleitstück in Längsrichtung eine weitere Durchbohrung (98) aufweist.

7. Vorrichtung nach Anspruch 5 und 6, dadurch gekennzeichnet, daß in der Durchbohrung (98) eine Nut (100) geformt ist, in der das Druckelement (101) zu Montagezwecken längsverschieblich geführt ist.

8. Vorrichtung nach einem der Ansprüche 1 bis 4, dadurch gekennzeichnet, daß im Gleitstück mindestens eine Kugelpfanne geformt ist zur Aufnahme einer Kugel (111, 112, 124, 125), die in einer Durchbohrung (113, 114) die Knochenschraube aufnimmt, das Gleitstück mit Klemmmitteln (123, 128) versehen ist zur Festlegung der Kugel in einer eingestellten Lage und an der Kugel Feststellmittel angreifen zur Feststellung der Knochenschraube in einer gewünschten axialen Lage in der Kugel.

9. Vorrichtung nach Anspruch 8, dadurch gekennzeichnet,daß das Gleitstück zweiteilig ausgeführt ist zur Bildung einer geteilten Kugelpfanne und die Gleitstückabschnitte mindestens mit Hilfe einer Schraube gegeneinander verspannt werden.

10. Vorrichtung nach Anspruch 8, dadurch gekennzeichnet, daß das Gleitstück (120) auf einer Seite eine längliche Aussparung aufweist, in der eine Spannplatte (123) eingesetzt ist und die Spannplatte (123) mit mindestens einer Schraube (128) gegen mindestens eine Kugel (124, 125) spannbar ist.

11. Vorrichtung nach einem der Ansprüche 1 bis 10, dadurch gekennzeichnet, daß die Kugelhälften (111, 112) der geteilten Lagerkugel eine Umfangsnut (115) aufweist zur Aufnahme einer die Kugelhälften zusammenhaltenden Ringfeder (116).

12. Vorrichtung nach Anspruch 11, dadurch gekennzeichnet, daß die Ringfeder (116) zwei radial nach außen weisende Vorsprünge (117) aufweist, die mit Ausnehmungen im Gleitstück zusammenwirken und die Kugel in ihrer Lage halten, jedoch ein begrenztes Verschwenken der Kugel erlaubt.

13. Vorrichtung nach einem der Ansprüche 1 bis 12, gekennzeichnet durch ein an die Innenkontur der Trägerstange (91 bis 94) angepaßtes in das Ende der Trägerstange einsetzbares Kupplungsstück zur starren Verbindung von zwei Trägerstangen miteinander.

14. Vorrichtung nach einem der Ansprüche 1 bis 13, gekennzeichnet durch ein an die Innen- oder Außenkontur angepaßtes mit dem Ende einer Trägerstange starr verbindbares Anschlußstück (130).

15. Vorrichtung nach Anspruch 14, dadurch gekennzeichnet, daß an dem freien Ende des Anschlußstücks (130) eine Gelenkkugel (132) angebracht ist, die mit einer klemmend wirkenden Kugelpfanne zusammenwirkt.

16. Vorrichtung nach Anspruch 14, dadurch gekennzeichnet, daß am freien Ende des Anschlußstücks (130) ein Schaft (133) angebracht ist, vorzugsweise mit strukturierter Oberfläche.

17. Vorrichtung nach einem der Ansprüche 1 bis 16, dadurch gekennzeichnet, daß ein Verbindungsglied (70) zwischen den Trägerstangen zwei hintereinander angeordnete Kugelgelenke (52, 53; 82, 83) aufweist.

18. Vorrichtung nach Anspruch 17, dadurch gekennzeichnet, daß ein Verbindungsglied (70) zwei in Abstand voneinander angeordnete Kugelpfannen (73) aufweist, in denen die mit Verbindungsstäben (51, 81) verbundenen Kugeln (52, 82) klemmend befestigbar sind.

19. Vorrichtung nach Anspruch 18, dadurch gekennzeichnet, daß das Verbindungsglied (70) zwei Abschnitte (71) mit je zwei Kugelpfannenhälften aufweist und die beiden Abschnitte (71) durch eine Klemmschraube (72) gegeneinander und auf die Kugeln (52, 82) preßbar sind.

20. Vorrichtung nach einem der Ansprüche 15 bis 19, dadurch gekennzeichnet, daß die Kugeln über einen Verbindungsstab (51, 81) lösbar mit dem Anschlußstück verbunden sind und der Verbindungsstab an den Enden einer Trägerstange (50, 80) befestigbar ist.

21. Vorrichtung nach einem der Ansprüche 15 bis 20, dadurch gekennzeichnet, daß an der Gelenkpfanne (150) eine Anschlußstange (151) angebracht ist.

22. Vorrichtung nach einem der Ansprüche 17 bis 21, dadurch gekennzeichnet, daß mit einer Gelenkkugel (163) ein Klemmkörper (160) verbunden ist, auf dessen der Kugel (163) abgewandter Seite ein Gewindestift (165) angebracht ist, auf dem Gewindestift (165) ein zweiter ringförmiger Klemmkörper (166) verschiebbar angeordnet ist, beide Klemmkörper (160, 166) mittels einer auf den Gewindestift (165) geschraubten Mutter (169) gegeneinander preßbar sind und die einander zugekehrten Flächen der Klemmkörper (160, 166) zusammenwirkende Nuten (161, 162, 167, 168) aufweisen zur Aufnahme einer Knochenschraube.

23. Vorrichtung insbesondere nach Anspruch 1, dadurch gekennzeichnet, daß auf der Außenseite der Trägerstange (3) verschiebbare Haltevorrichtungen (2) die Trägerstange (3) zumindest teilweise umgreifen, die mittels einer Feststellschraube (9) in ihrer axialen Lage festlegbar sind und die Haltevorrichtung (2) eine Querbohrung aufweist zur Aufnahme eines die Klemmvorrichtung (4) haltenden Schaftes (6) ,der seinerseits in der Querbohrung festlegbar ist.

24. Vorrichtung nach Anspruch 23, dadurch gekennzeichnet, daß die Haltevorrichtung (2) aus je zwei C-förmigen Backenhälften (2a, 2b) besteht, deren Schenkel (23, 24) verschieden ausgebildet sind und daß die Backenhälften um die Trägerstange (3) legbar und durch eine Stellschraube (9) derart gegeneinander verspannbar sind, daß bei angezogener Stellschraube (9) die Haltebacke (2) die Trägerstange (3) umfaßt und auf dieser fixiert wird.

25. Vorrichtung nach Anspruch 24, dadurch gekennzeichnet, daß die Backenhälften (2a, 2b) durch eine Halteschraube (13) derart miteinander verschraubbar sind, daß in gelöstem Zustand der Stellschraube (9) die Haltebacke (2) auf der Trägerstange (3) frei verschiebbar aber gegen Verdrehung und Kippen gesichert ist.

26. Vorrichtung nach einem der Ansprüche 23 bis 25,dadurch gekennzeichnet, daß der mit einer Klemmbacke (4) verbundene Schaft (6) mit einer Feinprofilierung versehen und mittels einer Stellschraube (10, 40) in der Querbohrung (8, 38) fixierbar ist.

27. Vorrichtung nach einem der Ansprüche 23 bis 25, dadurch gekennzeichnet, daß der Schaft (6) der Klemmbacke (4) und die Querbohrung (8, 38) mit ineinandergreifenden, verdrehungssicheren Profilen, insbesondere mit Feinverzahnungen, versehen sind.

28. Vorrichtung nach einem der Ansprüche 23 bis 27,dadurch gekennzeichnet, daß zwischen der Klemmbacke (4) und dem Schaft (6) ein fixierbares Kippgelenk (7) angeordnet ist, dessen Kippachse quer zur Längsachse des Schaftes (6) verläuft.

29. Vorrichtung nach einem der Ansprüche 23 bis 28, dadurch gekennzeichnet, daß an dem der Klemmvorrichtung (4) abgewandten Ende (12) des Schaftes (6) Befestigungsmittel zum Befestigen einer weiteren Klemmvorrichtung vorgesehen sind.

30. Vorrichtung nach einem der Ansprüche 1 bis 29, dadurch gekennzeichnet, daß mit dem Gleitstück oder der Haltevorrichtung ein am Trägerrohr angebrachtes Kraftglied gekoppelt ist, das permanent oder alternierend eine einstellbare axiale Kraft auf das Gleitstück oder die Haltevorrichtung ausübt.

Fig. 1

Fig. 3

Fig. 2

# Fig. 4

# Fig. 5

# Fig. 6

EP 0 314 021 A2

**Fig. 7**

**Fig. 8**

**Fig. 9**

**Fig. 10**

# Fig. 11

# Fig. 12

# Fig. 13

# Fig. 14

# Fig. 15

Fig. 16

Fig. 17

Fig. 18

Fig. 19

Fig. 20

Fig. 21

Fig. 22

Fig. 23

Fig. 24

Fig. 25

Fig. 26

Fig. 27

Fig. 28

Fig. 29